# EUROPEAN PATENT APPLICATION

(11) **EP 3 926 053 A1**
(43) Date of publication of application: **22.12.2021**
(21) Application number: 21180414.1
(22) Date of filing: 18.06.2021
(51) Int. Cl.: C12Q 1/6806, C12Q 1/6851, C12Q 1/689

(54) **METHOD FOR DETERMINING THE CONCENTRATION OF A BACTERIUM OF INTEREST IN AN ENVIRONMENTAL MATRIX**

(30) Priority: 18.06.2020 IT 202000014683
(71) Applicant: Acea Elabori S.p.A. Gruppo ACEA S.p.A., 00189 Roma RM (IT)
(72) Inventor: Ottaviano, Claudio, Rome (IT)
(74) Representative: IP Sextant s.r.l.

(57) **Abstract**

Method (1) for determining the concentration of a bacterium of interest in one environmental matrix, the method comprising the following operational steps: A. Preparing at least one primary suspension (2) of one sample of the environmental matrix, thereby obtaining the breaking down of the particles of the environmental matrix and the separation of the bacterium of interest, if present, of the environmental matrix; B. Preparing one or more dilutions (21, 22, 23) of the primary suspension (2) in respective first containers (31, 32, 33); C. Preparing second containers (401, 402, 403; 411, 412, 413; 421, 422, 423; 431, 432, 433; 441, 442, 443) containing the at least one primary suspension (2) and said one or more dilutions (21, 22, 23); D. extracting the genome of the content of each second container (401, 402, 403; 411, 412, 413; 421, 422, 423; 431, 432, 433; 441, 442, 443) into a respective third container (501, 502, 503; 511, 512, 513; 521, 522, 523; 531, 532, 533; 541, 542, 543); E. Preparing, at least one plurality of tubes (601, 602, 603; 611, 612, 613; 621, 622, 623; 631, 632, 633; 641, 642, 643), each tube of the plurality of tubes containing the genome contained in a respective third container (501, 502, 503; 511, 512, 513; 521, 522, 523; 531, 532, 533; 541, 542, 543); F. Applying at least one qualitative analysis procedure to each tube (601, 602, 603; 611, 612, 613; 621, 622, 623; 631, 632, 633; 641, 642, 643), the qualitative analysis procedure being configured for detect the presence or absence of the genome of the bacterium of interest, and outputting for each tube (601, 602, 603; 611, 612, 613; 621, 622, 623; 631, 632, 633; 641, 642, 643) a corresponding index (101, 102, 103; 111, 112, 113; 121, 122, 123; 131, 132, 133; 141, 142, 143) of the presence or absence of the genome of the bacterium of interest; G. Based on each index (101, 102, 103; 111, 112, 113; 121, 122, 123; 131, 132, 133; 141, 142, 143) of the presence or absence of the genome of the bacterium of interest, applying a quantitative analysis procedure, the quantitative analysis procedure being configured to determine the value of the most probable number of said bacteria of interest in the at least one sample of environmental matrix.

## Description

The present invention relates to a method for determining the concentration of a bacterium of interest in an environmental matrix. More particularly, the present invention relates to a method particularly suitable to be used for determining, for example, the concentration of the Salmonella spp. bacterium in an environmental matrix of dehydrated mud (for example having about 80% humidity), deriving from the waste water purification process, or for example the concentration of the Legionella spp. or Legionella pneumophila bacterium in an aqueous environmental matrix.

In the present description and in the following claims, the term "environmental matrix" means a set of complex structures of the states of matter (gaseous, liquid, solid) that make up a specific natural environment, such as for example dehydrated or dry mud or water.

In the following description, as will be noted, reference will be made mainly to applications of the method according to the invention, to determine the concentration of Salmonella spp. in an environmental matrix of dehydrated mud or of Legionella spp. or Legionella pneumophila in an environmental matrix of water. However, it must be borne in mind that the method of the present invention can be applied in any other context of microbiological analysis, wherein the concentration of these or other micro-organisms of interest in any other environmental matrix must be determined, always remaining within the scope of protection of the present invention as defined by the appended claims.

Being able to quantify the concentration of a bacterium of interest in an environmental matrix is extremely useful.

In waste water treatment plants, for example, mud is produced as a derivative of the purification process. The mud derived from this type of process can include pathogens that can be dangerous for human health and, usually, is then sent for disposal. Alternatively, the mud resulting from the purification of waste water can be used in agriculture or other sectors, for example in forestry, as long as it complies with stringent chemical-physical and microbiological parameters, at the end of certification analyses that are usually conducted in the laboratory.

Among the microbiological controls required by regulations for reuse of mud, the determination of the concentration of Salmonella spp. (all species) appears to be among the most important, given the pathogenic nature of this bacterium and the negative consequences it can have for human health.

Therefore, it is required to determine the concentration of this bacterium in a certain amount by weight of mud and, if this concentration exceeds a preset threshold value (currently set at 1000 MPN/gss - dry substance - for Salmonella spp.), the mud sample must be disposed of. Otherwise it can be used for other purposes, with no risk for health.

One of the currently most used methods for determining the concentration of a bacterium of interest in an environmental matrix, such as the aforementioned dehydrated mud, comprises the following operational steps:
(a) preparation of a primary suspension, starting from a sample of the environmental matrix under investigation - this step allows the release of the micro-organisms therein aggregated and the separation of the microbial clusters;
(b) preparation of a plurality of dilutions of the primary suspension in first containers;
(c) pre-enrichment of the primary suspension and its dilutions by inoculating a preset amount of the primary suspension and its dilutions into respective second containers, each containing a preset amount of a primary averagely selective enrichment broth - this step promotes growth or multiplication of micro-organisms, including the bacterium of interest possibly present, contained in the primary suspension or in its dilutions;
(d) enrichment of the primary suspension and its thus pre-enriched dilutions, by inoculating a preset amount thereof in a respective third container, each third container containing a preset amount of a secondary selective broth - this step favours the growth of the bacterium of interest and, instead, inhibits the growth of other micro-organisms;
(e) isolation of the bacterium of interest in one or more respective plates containing a suitable culture medium - this step comprises streaking with a suitable loop or isolating, in a known way, the contents of each third container on a corresponding specific and selective solid medium included in a respective plate, wherein colonies of the bacterium of interest, if present, can be isolated;
(f) confirmation of the presence of the bacterium of interest, in each culture medium of each plate, by performing biochemical and serological identification tests - this step allows to identify the colonies of the bacterium of interest in the culture medium of each plate; and
(g) quantification of the concentration of the bacterium of interest, in each culture medium of each plate, through the use of known counting techniques - this step allows to quantify the number of bacteria of interest in the analysed sample.

On the basis of the method described in steps (a) to (g) it is possible to quantify the concentration (in technical jargon "title") of Salmonella spp. present in the examined environmental matrix (i.e., in the dehydrated mud sample).

In the specific case, step (g) of quantitative analysis indicated above is traditionally based on the technique of counting in MPN (*Most Probable Number,* most probable number), reported for example in Istisan Reports 14/18 - ISS F 002B rev. 00 of the National Institute of Health, colonies of the bacterium of interest.

Salmonella bacterium is able to reproduce in a Selenite-cystine broth at 36 ± 1°C, in the Rappaport Vassiliadis selective enrichment broth at 42 ± 1°C and to produce typical colonies on isolation media such as Rambach agar, XLT4 agar, XLD agar at 36 ± 1°C, whereby:
- step (a) of preparation of the primary suspension comprises mechanically homogenizing a predetermined amount of dehydrated mud with a sterile peptone saline solution of a suitable type, for example as indicated in Istisan Reports 14/18 - ISS F 002B rev. 00 of the National Institute of Health;
- dilution step (b) includes the dilution, with preset amounts of sterile peptone saline solution, of preset amounts of primary suspension within the first separate containers, for example as indicated in Istisan Reports 14/18 - ISS F 002B rev. 00 of the National Institute of Health;
- pre-enrichment step (c) comprises the subsequent inoculation of preset amounts of primary suspension or its dilutions, in respective second containers containing an averagely selective culture medium, based on Selenite-cystine. As an alternative to Selenite-cystine, for example, a solution of sterile buffered peptone water can be used - this step favours the growth (multiplication) of the micro-organisms contained in the primary suspension and is performed following, for example, the indications of the Istisan Reports 14/18 - ISS F 002B rev. 00 of the National Institute of Health;
- enrichment step (d) comprises the enrichment of the contents of each of the second containers, in a respective selective medium included within a respective third container, the selective medium comprising Rappaport Vassiliadis broth, for example obtained as indicated in the Istisan reports 14/18 - ISS F 002B rev. 00 of the National Institute of Health, which inhibits the growth of non-target micro-organisms and promotes that of Salmonella spp.;
- step (e), comprises the isolation of the bacterium of interest possibly contained in each of the third containers, on plates comprising specific and selective suitable agar media, for example of Rambach agar, XLT4 agar, XLD agar, and similar, obtained as indicated in the Istisan Reports 14/18 - ISS F 002B rev. 00 of the National Institute of Health;
- confirmation step (f),comprises execution of tests, for example predictive known tests for Oxidase, Indole, Gram stain, biochemical identification tests and serological confirmation tests to confirm, for each plate, the presence or absence of Salmonella spp. as an organism to be detected; and
- quantification step (g) follows previous confirmation step (f), and includes the determination, for the analysed sample, of a characteristic number (NC) and the use of that characteristic number in the MPN counting technique (*Most Probable Number*), to determine the most probable number of bacteria of interest, for example using a table like the one shown in Figure 7.

The sequential execution of steps (a) to (g) described above allows to quantify the concentration of Salmonella spp. in the examined environmental matrix.

The number of dilutions and the dilution ratio of each of the subsequent dilutions of the primary suspension containing the environmental matrix sample to be analysed is indicated, for example, in the Istisan Reports 14/18 - ISS F 002B rev. 00 of the National Institute of Health, and takes into account the legal limit for the specific bacterium of interest, provided for in national legislation.

An entirely similar method to the one reported above can be provided *mutatis mutandis* for the determination of the concentration of Legionella spp. or Legionella pneumophila in a sample of an aqueous matrix and is for example disclosed by the UNI EN ISO 11731/2017 standard. Another culture method of the known art is the AFNOR IDX 33/06-06/19 validated method.

In this regard, it should be remembered that the Legionella spp. or Legionella pneumophila bacterium (briefly hereinafter also just Legionella) is a highly pathogenic bacterium, which can nest in water systems. Therefore, it becomes essential to be able to detect their presence extremely quickly, in order to avoid service interruptions for the completion of the necessary sanitation actions, and restore health safety conditions in the use of available water resources.

The above recalled traditional methods for determining, in general, the concentration of a bacterium of interest in an environmental matrix and, in particular, the concentration of the bacterium of Salmonella spp. in an environmental matrix of dehydrated mud, or of Legionella in an aqueous matrix, suffer for various drawbacks.

In fact, they provide for the statistical determination of the number of cultivable bacteria present in a sample (MPN/g or MPN/ml or UFC/ml) after a series of passages, for example in culture media that require physiological technical time intervals for development and growth of the bacteria of interest in each culture medium. These are, therefore, laborious methods, which require the implementation of a number of steps with predetermined deadlines over a period of time between 6 and 14 days, in order to obtain the final result. By requiring, among other things, execution times even longer than one week, for the implementation of these methods (for Legionella up to 14 days of execution), it is required that operators also work during holidays, if necessary, with an increase in implementation costs.

In addition, those traditional methods require a large amount of materials to be used, previously prepared by the laboratory, which occupy, among other things, a certain volume, and which must be properly disposed of, thus having an impact on the logistics of the analysis.

Not only that, the aforementioned traditional methods favour biological risk since, in order to be implemented, they require the multiplication of the (pathogenic) bacterium of interest.

In addition to the above traditional methods, other known methods for the detection and quantification of the concentration of a micro-organism of interest in an environmental matrix are described in US2004/241644 A1 and in EP1231280 A1. In document EP1231280 A1, in particular, it is taught to detect and quantify a micro-organism having a specific function, coming from a sample taken from a natural environment, by:
- one serial dilution of that sample and a subsequent incubation of the dilutions thereby obtained, in conditions which favour the growth of the aforementioned micro-organism having a specific function;
- one count, in each incubated serial dilution, of the micro-organism having a specific function by means of the MPN counting technique, and in parallel, one count of the total number of micro-organisms and the total number of heterotrophic micro-organisms, for example by direct counting under the microscope;
- the estimate of the dominant level of the micro-organism having a specific function in the natural environment, from the ratio between the number of micro-organisms having a specific function previously counted and the total number of micro-organisms and/or of heterotrophic micro-organisms previously counted as well;
- the selection, among the incubated serial dilutions, of the one with the highest dilution ratio, so that a growth of the micro-organism having a specific function, results and, then
- a subsequent step of extraction of the DNA of the aforementioned micro-organism of interest from this selected dilution, amplification by PCR of specific gene regions, by using said DNA as a reference, followed by cloning;
- examination of the differences of the gene regions thus cloned and determination of the related nucleotide sequences and the subsequent identification of the micro-organism with a specific function that populates the natural environment.

In EP1231280 A1 the growth of the micro-organism of interest in each incubated dilution is assessed by observing its turbidity or, for example, by observing the change in its pH or substrate concentration. It can also be confirmed by direct observation under the microscope.

These methods are laborious and take a long time to be carried out too.

Therefore, there is the need to provide for a method for determining the concentration of one bacterium of interest in an environmental matrix, which solves the aforementioned drawbacks.

More particularly, the object of the present invention is to provide for a method for determining the concentration of a bacterium of interest in an environmental matrix, which is faster than the traditional determination method.

Anther object of the present invention is to provide for a method for determining the concentration of a bacterium of interest in an environmental matrix, which is simpler than the traditional determination method.

Yet another object of the present invention is to provide for a method for determining the concentration of a bacterium of interest in an environmental matrix, which requires a smaller amount of materials for its execution and wherein the required materials occupy a smaller volume compared to the traditional determination method

A further object of the present invention is to provide for a method for determining the concentration of a bacterium of interest in an environmental matrix, which is less costly than the traditional determination method.

Not the least object of the present invention is to provide for a method for determining the concentration of a bacterium of interest which minimizes the biological risk.

It is a specific object of the present invention one method for determining the concentration of one bacterium of interest in one environmental matrix, the method comprising the following operational steps:
A. Preparing at least one primary suspension of one sample of said environmental matrix;
B. Preparing one or more dilutions of said at least one primary suspension in respective first containers;
C. Preparing second containers, containing said at least one primary suspension and said one or more dilutions;
D. Extracting the genome of the content of each second container into a respective third container;
E. Preparing at least one plurality of tubs, each tube of the plurality of tubes containing the genome contained in a respective third container;
F. Applying at least one qualitative analysis procedure to each tube, said qualitative analysis procedure being configured for detecting the presence or absence of the genome of said bacterium of interest, and outputting for each tube a corresponding index of the presence or absence of said genome of said bacterium of interest;
G. Based on each index of the presence or absence of said genome of said bacterium of interest, applying a quantitative analysis procedure, said quantitative analysis procedure being configured to determine the value of the most probable number of said bacteria of interest in said at least one sample of environmental matrix.

According to another aspect of the invention, said step A can comprise:
- if said environmental matrix is a solid one, mechanically homogenize a preset amount of said sample of said environmental matrix with a sterile peptone saline solution, or
- if said environmental matrix is an aqueous one, preliminary filtering a preset amount of said sample of said aqueous environmental matrix, by using at least one filter, putting the used filter for said filtration in one container already containing another preset amount of said sample of said aqueous environmental matrix and shaking for a preset shaking interval.

According to a further aspect of the invention, said step C can comprise inoculating one predefined amount of said primary suspension and of each subsequent dilution of said primary suspension, in one or more respective second containers.

According to an additional aspect of the invention, if said environmental matrix is a solid one, each container can contain at least one predefined amount of an averagely selective medium and said step C further comprises incubating each second container at a preset incubation temperature, for a defined incubation interval, so that in each second container the multiplication of said bacterium of interest, if present, is obtained.

According to another aspect of the invention, said step D can comprise, for each second container:
- transferring one predefined amount of said primary suspension or said dilution thereof into said respective third container; and
- adding to said third container one predefined amount of at least one lysis buffer,
   at a temperature at least equal or higher that a minimum temperature T_{MIN}, to break the cells therein contained; and
- centrifuging said at least one third container for at least one minimum time t_{MIN}.

According to another aspect of the invention, if said environmental matrix is an aqueous one, said step D, after transfer of said predefined amount of said primary suspension or said dilution thereof into said respective third container and before the addition in each third container of said predefined amount of at least one lysis buffer can comprise:
- adding into each third container a predefined amount of an enzyme configured to degrade the genome of dead cells possibly therein contained.

According to a further aspect of the invention, said step E of preparing said at least one plurality of tubes, can comprise:
- transferring a predefined amount of the content of each respective third container, optionally lower than 5 µl, more optionally equal to 3 µl, into a respective tube; and
- if not already present in each respective tube, adding at least one predefined amount of one amplification reagent of the genome of said bacterium of interest.

According to an additional aspect of the invention, said qualitative analysis procedure can comprise for each tube:
- waiting for a predetermined amplification time interval, during which the amplification of said genome of said bacterium of interest possibly therein contained, optionally by means of a loop-mediated isothermal amplification technique, occurs;
- applying a detection technique of said genome thus amplified, and outputting a respective index of presence or absence of said genome.

According to another aspect of the invention, said technique for detection of said genome can be a photometric technique that can comprise, for each tube:
- detecting during said predetermined amplification time interval, at least one fluorescent light radiation emitted from each tube, as a result of the amplification of the genome triggered at the end of said step E; and
- outputting one corresponding index of presence or absence of said genome, based on the fact that said at least one fluorescent light radiation emitted by each tube so detected exceeds or not at least one predefined threshold value or it has one determined time pattern or not.

According to a further aspect of the invention, said quantitative analysis procedure can comprise a statistical analytical technique of indirect counting of the most probable number of said bacteria of interest into said at least one sample of environmental matrix.

According to an additional aspect of the invention, said quantitative analysis procedure can comprise:
determining, on the basis of each index of presence or absence of said genome, one Characteristic Number, according to the MPN value (most probable number) counting technique; and
- analytically determining, optionally by querying a database or consulting a consultation table, the value of the most probable number of said bacteria of interest per gram of sample (MPN/g) and/or the most probable number of said bacteria of interest per gram (MPN/g ss) of dry matter if said environmental matrix is solid, or the most probable number of said bacteria of interest per ml of sample (MPN/ml) and/or the most probable number of said bacteria of interest per 100 ml (MPN/100 ml) of sample if said environmental matrix is aqueous, as a function of said Characteristic Number, of said dilution ratio and of said percentage weight of dry matter into said sample.

According to another aspect of the invention, said at least one bacterium of interest can be Salmonella spp. or Legionella spp. or Legionella pneumophila.

According to a further aspect of the invention, if said bacterium of interest is Salmonella spp., said step C can comprise inoculating, in respective second containers one preset amount of said at least one primary suspension or one preset amount of at least one dilution thereof into a predetermined amount of an averagely selective Selenite-cystine based medium and incubating at a temperature comprised between 36 ± 5 ° C, optionally comprised between 36 ± 4 ° C, more optionally between 36 ± 1 ° C for 21 ± 3 hours.

According to an additional aspect of the invention, if said bacterium of interest is Salmonella spp. in said step D the cellular lysis of the content of said at least one third container can be carried out for at least one minimum lysis time interval tₘᵢₙ, optionally between 25 and 35 min, more optionally equal to 30 min at a temperature at least equal to or greater than a minimum temperature T_{MIN}, dove T_{MIN} is optionally equal to 60°C, more optionally equal to 65°C and the centrifugation of said at least one third container is performed for a minimum time interval t_{MiN} optionally lower than 10 minutes, more optionally equal to 5 minutes, at a speed between 7500/rpm and 9000/rpm, optionally equal to 8500 rpm.

According to another aspect of the invention, if said bacterium of interest is Legionella spp. or Legionella pneumophila, in said step D after the transfer of said predefined amount of said primary suspension or said dilution thereof into said third container and before addition into said at least one third container of said predefined amount of at least one lysis buffer, one amount of said enzyme configured to degrade the genome of the dead cells possibly therein contained, optionally comprised between 45 µl and 55 µl, more optionally equal to 50 µl, is added into said at least one third container and the cellular lysis of the content of said at least one third container is carried out for at least one minimum lysis time interval tₘᵢₙ, optionally between 10 and 20 min, more optionally equal to 15 min at a temperature at least equal to or greater than a minimum temperature T_{MIN}, wherein T_{MIN} is optionally equal to 90°C, more optionally equal to 95°C and wherein the centrifugation of said at least one third container is performed for a minimum time interval t_{MIN} optionally lower than 10 minutes, more optionally equal to 5 minutes, at a speed between 7500/rpm and 9000/rpm, optionally equal to 8500 rpm.

The present invention will be now described, for illustrative but not limiting purposes, according to its preferred embodiments, with particular reference to the Figures of the accompanying drawings, wherein:

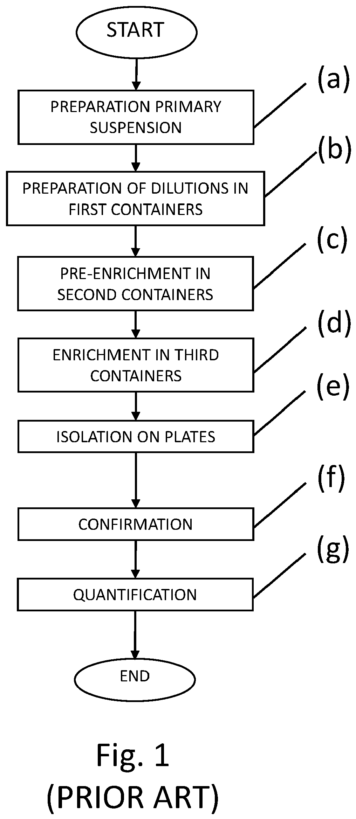

Figure 1 shows the main steps of a method for determining the concentration of a bacterium of interest in an environmental matrix according to the known art;
Figure 2 is a representation of the main steps of a method for determining the concentration of a bacterium of interest in an environmental matrix, according to the present invention;
Figures 3a to 3e illustrate the material and part of the instrumentation that can be used in the implementation of the method according to a first embodiment of the present invention, when the environmental matrix wherein the concentration of the bacterium of interest is to be determined is solid;
Figure 4 is a reference table, which can be used in the implementation of the method according to the aforementioned first embodiment of the present invention; and
Figure 5 shows part of the material that can be used in the method according to a second embodiment of the present invention, when the environmental matrix wherein the concentration of the bacterium of interest is to be determined is aqueous.

In the enclosed Figures the same reference numerals will be used for similar elements.

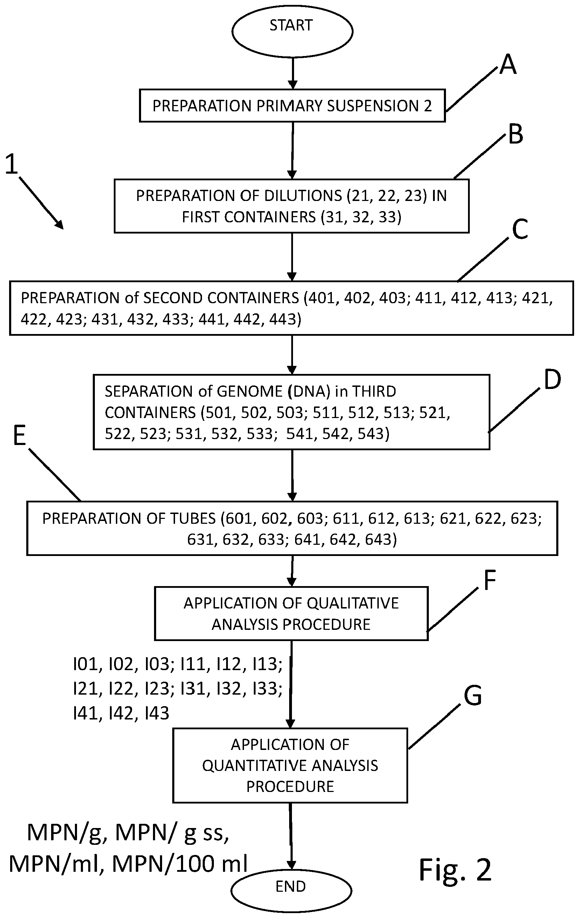

With reference now to Figures 2 to 5, it will be noted how the method for determining the concentration of a bacterium of interest in an environmental matrix according to the present invention is indicated with the reference number 1 and comprises a first operating step A of preparation of a primary suspension 2, starting from a sample of an environmental matrix. In this step, according to the first embodiment of the invention, when the sample of the environmental matrix is solid, for example dehydrated mud, the disintegration of the particles of the sample of the environmental matrix is obtained as well as the separation of the bacterium of interest, if present in the sample, from the remaining part of the environmental matrix.

In the specific case of the research of the bacterium of Salmonella spp. in one sample of environmental matrix of dehydrated mud, deriving from purification of wastewater, step A comprises mechanically homogenizing a preset amount of environmental matrix with a sterile peptone saline solution, for example according to the amounts indicated in Istisan Reports 14/18 - ISS F 002B rev. 00 of the National Institute of Health or according to other preestablished ratios. According to a preferred embodiment of the invention, for example, 10-30 g of dehydrated mud, optionally 20 g, can be homogenized with 150-200 ml, optionally 180 ml, of sterile peptone saline solution.

The aforementioned step A, in the case wherein the environmental matrix is instead aqueous, foresees the preliminary filtering of a predefined amount of a sample of the aforementioned environmental matrix, using (for example filtration ramps and) at least one filter, and putting the filter used for filtration in a container already containing a predetermined amount of that environmental matrix sample. Step A subsequently comprises the agitation of the container for a predefined agitation time. In the specific case of Legionella quantification, for example, step A of the method according to the invention requires the filtration of about 90 ml of an aqueous environmental matrix sample, using at least one filter with porosity between 0.40 µm and 0, 50 µm, optionally equal to 0.45 µm, and with a diameter in PES (polyethersulfone) between 45 mm and 49 mm, optionally equal to 47 mm. The filter is then placed in a sterile container containing about 10 ml of the environmental matrix sample of water to be analysed and then an agitation of the container for 15 minutes in an orbital shaker at 250 rpm follows.

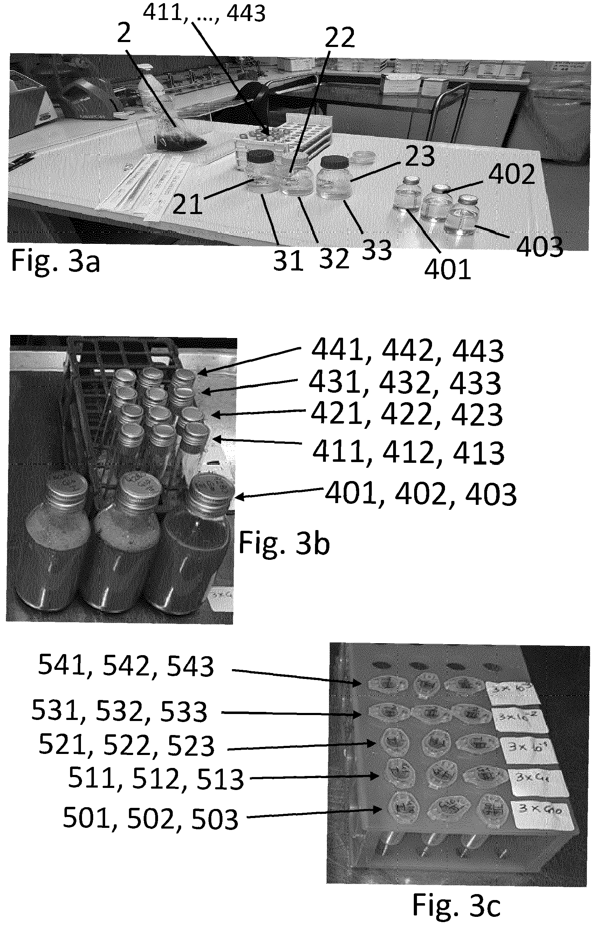

Once the primary suspension 2 is obtained, the method 1 of the present invention comprises a dilution step B of the primary suspension 2 obtained in step A, wherein one or more subsequent dilutions (21, 22, 23) of the primary suspension 2 are prepared in corresponding first containers (31, 32, 33), each subsequent dilution (21, 22, 23) having a respective dilution ratio (R1, R2, R3) with respect to the primary suspension 2 (see in particular Figure 3a).

According to a preferred variant of the invention, the dilution ratios R1, R2, R3 with respect to the primary suspension 2 are decreasing decimals, for example respectively equal to 10⁻¹, 10⁻², 10⁻³, even if, depending on the case, the primary suspension 2 could be further diluted and/or according to completely different dilution ratios therefrom. For example, the dilution ratio could be 1 to 2 between one dilution and the next one. The preparation of each subsequent dilution (21, 22, 23) comprises inoculating in the respective first container (31, 32, 33) a predetermined amount of primary suspension 2 in a predetermined amount of sterile peptone saline solution, so that at the end of dilution step B, first containers (31, 32, 33) are obtained, each containing a different concentration of primary suspension 2.

Purely by way of example, according to the first embodiment of the invention, wherein the sample of the environmental matrix is solid, for example dehydrated mud, three dilutions (21, 22, and 23) of the primary suspension 2 can be obtained, with dilution ratios R1= 10⁻¹, R2= 10⁻², R3= 10⁻³ as follows:
- a first dilution 21 with dilution ratio R1 is obtained by taking 10 ml of primary suspension 2 and pouring them into a first container 31 containing 90 ml of sterile peptone saline solution,
- a second dilution 22 with dilution ratio R2 is obtained by taking 10 ml from the first container 31, containing the first dilution 21 with dilution ratio R1, and pouring them into another first container 32 containing 90 ml of sterile peptone saline solution, and
- a third dilution 23 with dilution ratio R3 is obtained by taking 10 ml from this latter first container 32 containing the second dilution 22, with dilution ratio R2, and pouring them into one further first container containing 90 ml of sterile peptone saline solution.

Clearly, in order to obtain further dilutions, one can proceed in the same way, taking for example 10 ml from a container containing the last dilution of the primary suspension, i.e. the one in which the dilution ratio is greater than the dilution ratios of the other dilutions, and adding it to another first container containing 90 ml of sterile peptone saline solution.

According to the other embodiment of the invention, in the case wherein the environmental matrix is instead aqueous, at step B, three dilutions (21, 22, and 23) of the primary suspension 2 can be obtained, with dilution ratios R1= 10⁻¹, R2= 10⁻², R3= 10⁻³ as follows:
- a first dilution 21 with dilution ratio R1 is obtained by taking 1 ml of primary suspension 2 and pouring it into a first container 31 containing 9 ml of sterile peptone saline solution,
- a second dilution 22 with dilution ratio R2 is obtained by taking 1 ml from the first container 31, containing the first dilution 21 with dilution ratio R1, and pouring it into another first container 32 containing 9 ml of sterile peptone saline solution; and
- a third dilution 23 with dilution ratio R3 is obtained by taking 1 ml from this latter first container 32, containing the second dilution 22 with dilution ratio R2, and pouring it into a further first container containing 9 ml of sterile peptone saline solution.

Clearly, in order to obtain further dilutions, one can proceed in the same way, taking for example 1 ml from a container containing the last dilution of the primary suspension, i.e. the one in which the dilution ratio is greater than the dilution ratios of the other dilutions, and adding it to another first container containing 9 ml of sterile peptone saline solution. Then, method 1 of the present invention comprises one step C for the preparation of second containers, wherein a predefined amount of the primary suspension 2 and of each subsequent dilution (21, 22, 23) of the primary suspension 2 is inoculated, in one or more respective second containers (401, 402, 403; 411, 412, 413; 421, 422, 423; 431, 432, 433; 441, 442, 443).

According to the first embodiment of the invention wherein the sample of the environmental matrix is solid, specifically dehydrated mud, step C foresees to inoculate a predefined amount of the primary suspension 2 and of each subsequent dilution (21, 22, 23) of the primary suspension 2, in one or more respective second containers (401, 402, 403; 411, 412, 413; 421, 422, 423; 431, 432, 433; 441, 442, 443) wherein each container contains at least one predefined amount of averagely selective medium. Step C then provides for leaving each of the aforementioned second containers (401, 402, 403; 411, 412, 413; 421, 422, 423; 431, 432, 433; 441, 442, 443) to incubate at a certain temperature for a certain time - see Figure 3b for reference which illustrates the second containers with the primary suspension and its pre-enriched dilutions after incubation.

By way of example, in the specific case in which the bacterium of interest is Salmonella spp., step C of preparation of the second containers includes:
- inoculating 10 ml of primary suspension 2 as it is (T.Q.) in a plurality of, optionally three, second containers (401, 402, 403), each containing 90 ml of medium selective medium, optionally comprising a Selenite-cystine broth;
- inoculating 1 ml of primary suspension 2 as it is (T.Q.) in a plurality of, optionally three, second containers (411, 412, 413), each containing 9 ml of averagely selective medium, optionally comprising a Selenite-cystine broth;
- inoculating 1 ml of the first suspension 21, diluted according dilution ratio R1, in a plurality of, optionally three, second containers (421, 422, 423), each containing 9 ml of averagely selective medium, optionally comprising a Selenite-cystine broth;
- inoculating 1 ml of the second suspension 22, diluted according dilution ratio R2, in a plurality of, optionally three, second containers (431, 432, 433), each containing 9 ml of averagely selective medium, optionally comprising a Selenite-cystine broth;
- inoculating 1 ml of the third suspension 21, diluted according dilution ratio R3, in a plurality of, optionally three, second containers (441, 442, 443), each containing 9 ml of averagely selective medium, optionally comprising a Selenite-cystine broth;
- incubating the second containers (401, 402, 403; 411, 412, 413; 421, 422, 423; 431, 432, 433; 441, 442, 443) containing the primary suspension 2 or the diluted suspension (21, 22, 23) thus pre-enriched at a temperature between 36 ± 5°C, optionally between 36 ± 4°C, more optionally between 36 ± 1°C for 21 ± 3 hours.

In the second embodiment of the invention, in the case that the environmental matrix is instead aqueous, step C of preparation of the second containers comprises:
- inoculating 1 ml of primary suspension 2 as it is (T.Q.) in a plurality of, optionally three, second containers (401, 402, 403);
- inoculating 1 ml of the first suspension 21, i.e. diluted according dilution ratio R1, in a plurality of, optionally three, second containers (411, 412, 413);
- inoculating 1 ml of the second suspension 22, i.e. diluted according dilution ratio R2, in a plurality of, optionally three, second containers (421, 422, 423); and
- inoculating 1 ml of the third suspension 21, i.e. diluted according dilution ratio R3, in a plurality of, optionally three, second containers (431, 432, 433).

Advantageously, unlike the traditional method, method 1 of the present invention then comprises one step D of extracting the genome contained in each second container (401, 402, 403; 411, 412, 413; 421, 422, 423; 431, 432, 433; 441, 442, 443).

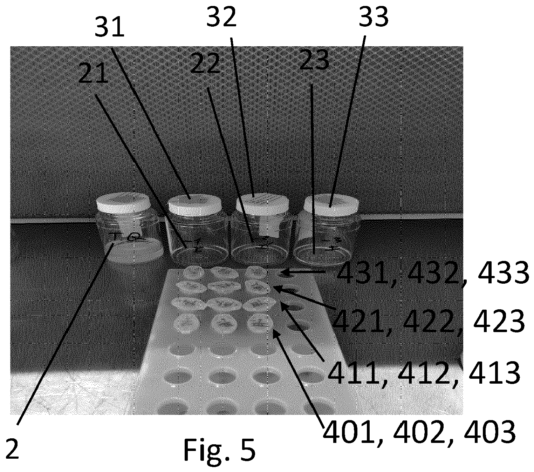

More particularly, step D comprises (see Figure 5):
- transferring a predefined amount, optionally less than 2 ml (depending on the amounts available in the second containers), more optionally equal to about 1 ml, of primary suspension 2 or diluted primary suspension (21, 22, 23) contained in one second container (401, 402, 403; 411, 412, 413; 421, 422, 423; 431, 432, 433; 441, 442, 443) in a respective third empty and sterile container (501, 502, 503; 511, 512, 513; 521, 522, 523; 531, 532, 533; 541, 542, 543);
- adding to each third container (501, 502, 503; 511, 512, 513; 521, 522, 523; 531, 532, 533; 541, 542, 543) one predefined amount of at least one lysis buffer, optionally lower than 2 ml, more optionally equal to about 1 ml, to break the cells therein contained; and
- centrifuging, in order to separate the supernatant, which includes the genome, from the pellet, which contains cellular debris and material of various kinds possibly present in each third container (501, 502, 503; 511, 512, 513; 521, 522, 523; 531, 532, 533; 541, 542, 543).

Step D ends by obtaining the separation of the genome possibly present in each third container (501, 502, 503; 511, 512, 513; 521, 522, 523; 531, 532, 533; 541, 542, 543) from cell debris.

According to one variant of the present invention, the addition of the lysis buffer and centrifugation can be performed in succession. Lysis, at a temperature at least equal to or higher than a minimum temperature (T_{MIN}) for at least one minimum lysis time interval (tₘᵢₙ), in a thermostatic bath, and centrifugation for at least one minimum time interval (t_{MIN}).

It is also specified that the amounts of primary suspension 2 or diluted primary suspension (21, 22, 23) transferred to the respective third empty and sterile containers (501, 502, 503; 511, 512, 513; 521, 522, 523; 531, 532, 533; 541, 542, 543) and the amounts of lysis buffers respectively added can also be different from those indicated above, provided that ratio 1:1 is maintained therebetween.

In the specific case of the first embodiment of the invention, for the determination of the concentration of the Salmonella spp. bacterium, step D comprises the addition to each third container (501, 502, 503; 511, 512, 513; 521, 522, 523; 531, 532, 533; 541, 542, 543) of at least one lysis buffer, of a type suitable for the purpose and commonly on the market, and cell lysis is performed for each third container (501, 502, 503; 511, 512, 513; 521, 522, 523; 531, 532, 533; 541, 542, 543) and occurs for at least one minimum lysis time interval of (tmin), optionally between 25 and 35 min, more optionally equal to 30 min, in a thermostatic bath at a temperature at least equal to or higher than a minimum temperature (T_{MIN}), where T_{MIN} is optionally equal to 60°C, more optionally equal to 65°C.

Subsequent centrifugation is performed for a minimum time t_{MIN} optionally less than 10 minutes, more optionally equal to 5 minutes, at a speed between 7500/rpm and 9000/rpm, optionally equal to 8500 rpm.

The step D described above is applied in a completely analogous way also to the second embodiment of the present invention, that is, in the case wherein the environmental matrix is aqueous. In this case step D comprises:
- transferring a predefined amount, optionally equal to about 1 ml of primary suspension 2 or diluted primary suspension (21, 22, 23) contained in a second container (401, 402, 403; 411, 412, 413; 421, 422, 423; 431, 432, 433) in a respective third empty and sterile container (not shown in the figures);
- adding to each third container a predefined amount, optionally equal to about 1 ml, of at least one lysis buffer, to break up the cells contained therein; and
- centrifuging, in order to separate the supernatant, which includes the genome, from the pellet, which contains cellular debris and material of various kinds possibly present in each third container.

Step D ends by obtaining the separation of the genome possibly present in each third container from cellular debris.

Optionally, in the case of aqueous environmental matrix, after the transfer of the predefined amount of primary suspension 2 or of diluted primary suspension into the respective third empty and sterile container, and before the addition of the lysis buffer in each such third container, step D of method 1 of the present invention provides for adding in each third container a predefined amount of an enzyme (in technical jargon free removal cells), capable of degrading the genome of dead cells possibly contained therein. In this way, at the end of step D, the only genome possibly present in each third container is referable only to living cells (i.e. potentially pathogenic, since dead cells are not pathogenic).

In this case also, the addition of the lysis buffer and centrifugation can be performed in succession. Lysis, at a temperature at least equal to or higher than a minimum temperature (T_{MIN}) for at least one minimum lysis time interval (tmin), in a thermostatic bath, and centrifugation for at least one minimum time interval (t_{MIN}).

As mentioned above, also for the second embodiment of the invention the amounts of primary suspension 2 or of diluted primary suspension (21, 22, 23) transferred into the respective third empty and sterile containers and the amounts of the lysis buffers respectively added can also be different from those indicated above, provided that the ratio 1: 1 is maintained therebetween.

In the specific case of the second embodiment of the invention, for the determination of the concentration of the Legionella bacterium, cell lysis is carried out for at least one minimum lysis time interval (tmin), optionally between 10 and 20 min, more optionally equal to 15 min, in a thermostatic bath at a temperature at least equal to or greater than a minimum temperature (T_{MIN}), wherein T_{MIN} is optionally equal to 90°C, more optionally equal to 95°C. The following centrifugation is carried out performed for a minimum time interval t_{MIN} optionally lower than 10 minutes, more optionally equal to 5 minutes, at a speed between 7500/rpm and 9000/rpm, optionally equal to 8500 rpm.

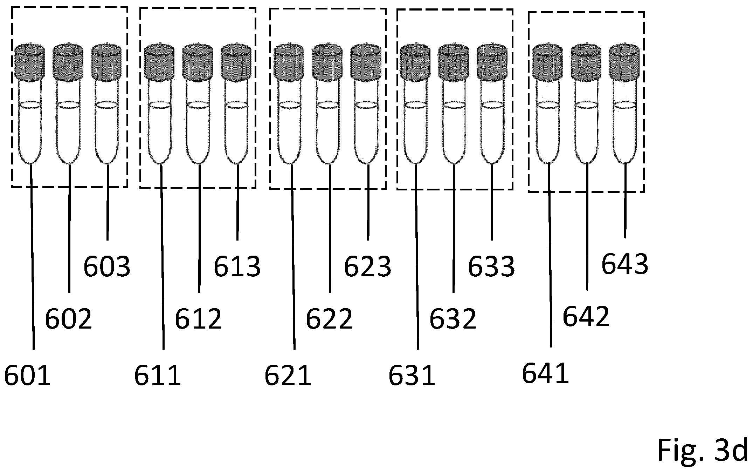

The execution parameters of step D reported above allow an optimal extraction of the genome present in the suspension contained in each third container (501, 502, 503; 511, 512, 513; 521, 522, 523; 531, 532, 533; 541, 542, 543), with respect to corresponding methods normally used for the extraction of the genome from a sample of a type similar to that of the present invention, which instead require shorter times and higher temperatures. Then, method 1 of the present invention comprises one step E (see Figure 3d) of preparing a plurality of tubes (601, 602, 603; 611, 612, 613; 621, 622, 623; 631, 632, 633; 641, 642, 643), each with a preset amount, optionally lower than 5 µl, more optionally equal to about 3 µl, of the contents of a respective third container (501, 502, 503; 511, 512, 513; 521, 522, 523; 531, 532, 533; 541, 542, 543). Each tube or test tube (601, 602, 603; 611, 612, 613; 621, 622, 623; 631, 632, 633; 641, 642, 643) also comprises at least one predetermined amount of another substance, i.e. at least one reagent (Primer in technical jargon) with which the genome of the bacterium of interest, if present in the content of the respective third container (501, 502, 503; 511, 512, 513; 521, 522, 523; 531, 532, 533; 541, 542, 543), mixes. According to one variant of the present invention, if in each tube or test tube (601, 602, 603; 611, 612, 613; 621, 622, 623; 631, 632, 633; 641, 642, 643) the aforementioned at least one reagent (primer in technical jargon) was not present, method 1 of the present invention would provide for adding a predetermined amount of such at least one reagent which, therefore, would get mixed with the genome of the bacterium of interest, if present in the content of the respective third container (501, 502, 503; 511, 512, 513; 521, 522, 523; 531, 532, 533; 541, 542, 543). According to the first embodiment, in the specific case of Salmonella spp., each tube or test tube (601, 602, 603; 611, 612, 613; 621, 622, 623; 631, 632, 633; 641, 642, 643) contains one ready-to-use set of reagents called Salmonella Screen Glow code EBT 615, produced by ENBIOTECH SRL and distributed by Avantech group SRL. With reference to the second embodiment, in the specific case of Legionella spp. or Legionella pneumophila in aqueous matrix, each tube or test tube (not shown in the Figures) contains one set of ready-to-use reagents called Enbiotech EBT 629 for Legionella spp. and Enbiotech EBT 621 for Legionella pneumophila. It is understood that other types of reagents, similar to those indicated above, could also be used.

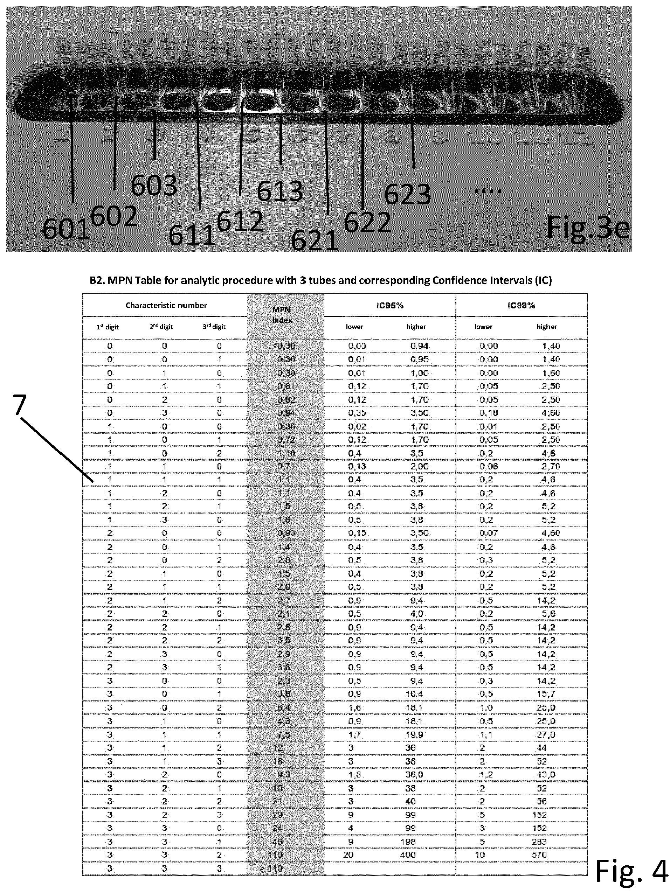

Next, the method of the present invention comprises a subsequent step F of applying at least one qualitative analysis procedure (see Figure 3e), configured to detect the presence or absence of the genome of the bacterium of interest in each tube or test tube (601, 602, 603; 611, 612, 613; 621, 622, 623; 631, 632, 633; 641, 642, 643) and independent of the type of environmental matrix analysed. At the end of this step, a corresponding index is obtained for each tube (601, 602, 603; 611, 612, 613; 621, 622, 623; 631, 632, 633; 641, 642, 643) (101, 102, 103; 111, 112, 113; 121, 122, 123; 131, 132, 133; 141, 142, 143) related to the presence ("+" sign in the following tables) or absence (" - "in the following tables) of the genome of the bacterium of interest in the examined tube.

According to a particularly advantageous aspect of the invention, the qualitative analysis procedure of method 1 of the present invention comprises the isothermal amplification (or Loop-mediated isothermal amplification or LAMP) of the genome of the bacterium of interest, possibly present in each tube (601, 602, 603; 611, 612, 613; 621, 622, 623; 631, 632, 633; 641, 642, 643), triggered at the end of the previous step E, when the contents of each third container (501, 502, 503; 511, 512, 513; 521, 522, 523; 531, 532, 533; 541, 542, 543) has been transferred to the respective tube (601, 602, 603; 611, 612, 613; 621, 622, 623; 631, 632, 633; 641, 642, 643) and mixed with the at least one reactant.

More specifically, this qualitative analysis procedure includes:
- waiting for a predetermined amplification time interval during which the amplification of the genome of the bacterium of interest contained in each tube takes place (601, 602, 603; 611, 612, 613; 621, 622, 623; 631, 632, 633; 641, 642, 643), triggered at the end of the previous step; and
- applying a technique for detecting the presence or absence of such a genome.

It should be noted that the above two steps of waiting for the predetermined amplification time interval and of applying the technique for detecting the presence or absence of such a genome can be carried out simultaneously.

According to a particularly advantageous aspect of the invention, the amplification of the genome of the bacterium of interest possibly present in each tube (601, 602, 603; 611, 612, 613; 621, 622, 623; 631, 632, 633; 641, 642, 643) occurs during an amplification time interval, optionally less than 90 minutes, more optionally equal to 60 minutes for Salmonella spp. and equal to 70 minutes for Legionella spp. or Legionella pneumophila.

Furthermore, the detection technique used in method 1 of the present invention can be a photometric technique, according to which a fluorescent light radiation emitted by each tube (601, 602, 603; 611, 612, 613; 621, 622, 623; 631, 632, 633; 641, 642, 643), due to the genome amplification triggered at the end of the previous stage of preparation of the tube itself, when the genome eventually extracted at step D is mixed with the at least one amplification reagent already present or added in the tube, is detected. The fluorescent light radiation is detected by means of a spectro-fluorometer and its time course is memorized.

Based on the fact that the respective fluorescent light radiation emitted, detected by the detector device, exceeds or not a predefined threshold value or assumes a predefined time course or not, at the output, for each tube (601, 602, 603; 611, 612, 613; 621, 622, 623; 631, 632, 633; 641, 642, 643) a corresponding index (I01, I02, I03; I11, I12, I13; I21, I22, I23; I31, I32, I33; I41, I42, I43) of presence or absence in the examined tube, of the genome of the bacterium of interest, is then supplied.

More specifically, during this step F, the intensity of the fluorescent radiation emitted by each tube (601, 602, 603; 611, 612, 613; 621, 622, 623; 631, 632, 633; 641, 642, 643) can have a sigmoid-like time course, if the amplification of the genome has occurred, with consequent emission of fluorescence - in this case the tube is "positive" (i.e. it contains the genome of interest). On the contrary, the trend of the intensity of the emitted fluorescent radiation can be of the linear type, if the amplification of the genome has not occurred (because the genome is not present in the examined tube) - in this case the tube is "negative" (i.e. it does not contain the genome of interest).

The method 1 of the present invention, then, advantageously comprises the application, based on each index (101, 102, 103; I11, 112, 113; 121, 122, 123; 131, 132, 133; 141, 142, I43) of the presence or absence of the genome of the bacterium of interest, determined in previous step F, of a quantitative analysis procedure (step G), configured to determine the value of the most probable number of bacteria of interest, per gram of sample (MPN/g) and/or the most probable number of bacteria of interest per gram (MPN/g ss) of dry matter, in the sample of environmental solid matrix examined, or the value of the most probable number of bacteria of interest per ml of sample (MPN/mL) and/or the most probable number of bacteria of interest per 100 mL (MPN/100 mL) of aqueous environmental matrix sample.

In this regard, the quantitative analysis procedure includes a statistical analytical technique of indirect counting of the most probable number of bacteria of interest in the examined sample. Specifically, it comprises:
- determining, based on each index (101, 102, 103; I11, 112, 113; 121, 122, 123; 131, 132, 133; 141, 142, 143) of presence or absence of that genome, one Characteristic Number (NC), according to the MPN value (most probable number) counting technique; and
- analytically determining, optionally by querying a database or consulting a consultation table 7, the value of the most probable number of said bacteria of interest per gram of sample (MPN/g) and/or the most probable number of said bacteria of interest per gram (MPN/g ss) of dry matter, or the most probable number of said bacteria of interest per ml of sample (MPN/ml) and/or the most probable number of said bacteria of interest per 100 ml (MPN/100 ml) of sample of said environmental matrix, as a function of said Characteristic Number (NC), of said dilution ratio (R1, R2, R3) and of said percentage weight of dry matter of examined sample, for example as indicated in the Istisan reports 14/18 - ISS F 002B rev. 00 of the National Institute of Health in the case of a solid environmental matrix.

By way of example only, details and results of some experimental tests carried out applying both the traditional method and the method 1 of the present invention for the determination of the concentration of Salmonella spp. in a sample of dehydrated mud, resulting from the purification of waste water, are provided below. Similar results are obtained from experimental tests carried out with the method of the present invention for the determination of the concentration of Legionella spp. or Legionella pneumophila in a sample of aqueous matrix (water).

### Example 1

### Traditional method

A preset amount equal to 20 gr of dehydrated mud sample deriving from the purification of wastewater was analysed according to the traditional method summarized in the introduction, for the determination of the concentration of Salmonella spp.

According to the traditional method, three successive decimal dilutions were obtained at step (b) - of the primary suspension obtained at step (a) - with respective dilution ratios (10⁻¹, 10⁻², 10⁻³) and the method was carried out up to step (e) with the attainment of respective plates containing, if present, colonies of the bacterium of Salmonella spp. in Rambach agar and XLT4 agar culture media. The number of plates in which the presence (sign "+" in the table below) or absence (sign "-" in the table below) of the Salmonella spp. bacterium was detected, by means of the aforementioned biochemical and serological identification tests, was then noted and the Characteristic Number (NC) was identified, considering that, as is known, it corresponds to a three-digit number, derived from the results of three successive dilutions (corresponding to the plates examined) having the highest dilution ratio, for which in at least one plate the presence of the bacterium of interest has been detected (in short, for which the number of positive plates is> 0).

According to the traditional method, it is reminded that the NC characteristic number must be determined, when possible, by choosing three successive dilutions for which the results are neither totally positive nor totally negative, i.e. for which not all the analysed plates detect the presence of the bacterium or not all the analysed plates detect the absence of the bacterium. If this is not possible, it is preferable to choose the three successive dilutions with the highest dilution ratio, wherein all the analysed plates detect the presence of the bacterium. If less than three successive dilutions show positive results (i.e., at least one plate in which the presence of the bacterium is detected), the dilution containing the highest concentration in the sample and the two subsequent dilutions must be considered for the determination of the characteristic number. If there are positive plates (in which the presence of the bacterium of interest is detected) for only one of the serial dilutions, that dilution with a positive result shall be considered, together with the previous and subsequent negative dilutions, to determine the NC characteristic number.

Table 1 below reports the results obtained in the experimental test conducted by applying the traditional method.

**Table 1**

| Sample dilution | T.Q. | | | T.Q. | | | 10⁻¹ | | | 10⁻² | | | 10⁻³ | | |
|---|---|---|---|---|---|---|---|---|---|---|---|---|---|---|---|
| | | | | | | | R1 | | | R2 | | | R3 | | |
| Inoculum (ml) | 10 | 10 | 10 | 1 | 1 | 1 | 1 | 1 | 1 | 1 | 1 | 1 | 1 | 1 | 1 |
| Selenite-cystine broth ml (pre-enrichment) | 90 | 90 | 90 | 9 | 9 | 9 | 9 | 9 | 9 | 9 | 9 | 9 | 9 | 9 | 9 |
| Rappaport Vassiliadis | + | + | + | + | + | + | + | + | + | + | + | + | + | + | + |
| Rambach agar | + | + | + | + | + | + | + | + | + | + | + | - | - | - | - |
| XLT4 agar | + | + | + | + | + | + | + | + | + | + | + | - | - | - | - |
| NC | 3 | | | 3 | | | 3 | | | 2 | | | 0 | | |

Based on he obtained results, it was determined that the NC is equal to 320. From here, according to the traditional method, to determine the concentration of bacterium in the examined sample, consultation table 7 was consulted, illustrated in Fig. 4, from which it was found that for an NC equal to 320, the corresponding MPN/g value is equal to 9,3. In order to express the result as MPN/g ss (dry substance), the dilution factor and the weight percentage % of the environmental matrix 2 were taken into account. In this specific case, with a dehydrated weight value of 20% and based on the results derived from the dilutions that were carried out, it was determined that the value in MPN/g ss is equal to 4650.

### Method according to the invention

According to the method of the present invention, a sample of primary suspension 2 was prepared containing 20 g of dehydrated mud deriving from the purification of waste water and 180 ml of sterile peptone saline solution (step A).

The method proceeded as described above with the preparation of three successive decimal dilutions - of the primary suspension obtained at step A - with respective dilution ratios (10⁻¹, 10⁻², 10⁻³) and with the preparation of the second pre-enriched containers (step C). Then, at step D the genome contained in the third containers was extracted, using a suitable lysis buffer and, by incubating in third containers at a temperature of 65°C for 30 minutes in a thermostatic bath, the cells breaking and genome leaking from the cellular compartment was obtained. With the subsequent centrifugation the genome was separated from the rest of the material which deposited, instead, on the bottom of the third containers, in the form of sediment (called pellet in technical jargon).

The tubes (601, 602, 603; 611, 612, 613; 621, 622, 623; 631, 632, 633; 641, 642, 643) were then prepared at step E, as described above and the procedure of qualitative analysis above (step F) was carried out, accordingly the genome of Salmonella spp. possibly contained in each tube was amplified by LAMP technology and its presence or absence was also detected by means of a system for molecular biology analysis (the ICGENE mini Cat. No. EBT 801, produced by ENBIOTECH SRL).

Subsequently, the quantitative analysis procedure described above was applied (step G), wherein the NC characteristic number was determined, advantageously based on the results of the qualitative analysis procedure, thus obtaining the value of MPN/g or MPN/g ss.

Table 2 below reports the results obtained in the experimental test conducted by applying the method of the present invention.

**Table 2**

| Sample dilution | T.Q. | | | T.Q. | | | 10-1 | | | 10⁻² | | | 10-3 | | |
|---|---|---|---|---|---|---|---|---|---|---|---|---|---|---|---|
| Tube: | 601 | 602 | 603 | 611 | | 612 | 621 | | 622 | 631 | | 632 | 641 | | 642 |
| | | | | 613 | | | 623 | | | 633 | | | 643 | | |
| Inoculum (ml) | 10 | 10 | 10 | 1 | 1 | 1 | 1 | 1 | 1 | 1 | 1 | 1 | 1 | 1 | 1 |
| Selenite broth ml (pre-enrichment) | 90 | 90 | 90 | 9 | 9 | 9 | 9 | 9 | 9 | 9 | 9 | 9 | 9 | 9 | 9 |
| LAMP | + | + | + | + | + | + | + | + | + | + | + | - | - | - | - |
| | 101 | 102 | 103 | 111 | 112 | 113 | 121 | 122 | 123 | 131 | 132 | 133 | 141 | 142 | 143 |
| NC | 3 | | | 3 | | | 3 | | | 2 | | | 0 | | |

In this case also an NC is derived, and it is equal to 320, to which a value of MPN/g equal to 9,3 is associated, based on what is reported in the consultation table 7. Taking into account the dilution and the dehydrated weight of the mud at 20% in the starting sample, the value in MPN/g ss is equal to 4650.

As is evident, then, method 1 of the present invention provides the same result as the traditional method in the same unit of measurement MPN/g ss.

### Example 2

With the traditional method and with the method of the present invention described above for the quantification of the concentration of Salmonella spp. ten (10) samples of dehydrated mud resulting from waste water purification were analysed.

Table 3 and the histogram below show the obtained results.

**Table 3**

| n. | Sample | Traditional method | | | Method of the invention | | |
|---|---|---|---|---|---|---|---|
| | | NC | MPN/g | MPN/g ss | NC | MPN/g | MPN/g ss |
| 1 | 25430 | 320 | 9.3 | 375 | 320 | 9.3 | 375 |
| 2 | 25584 | 000 | 0 | 0 | 100 | 0.36 | 1.48 |
| 3 | 25193 | 000 | 0 | 0 | 000 | 0 | 0 |
| 4 | 26024 | 000 | 0 | 0 | 000 | 0 | 0 |
| 5 | 26286 | 300 | 23 | 100 | 300 | 23 | 100 |
| 6 | 26384 | 100 | 0.36 | 1.48 | 100 | 0.36 | 1.48 |
| 7 | 26776 | 321 | 15 | 72 | 322 | 21 | 101 |
| 8 | 27006 | 320 | 9.3 | 284 | 321 | 150 | 458 |
| 9 | 27368 | 110 | 0.71 | 3.94 | 010 | 0.3 | 1.67 |
| 10 | 27456 | 100 | 0.36 | 2.06 | 100 | 0.36 | 2.06 |

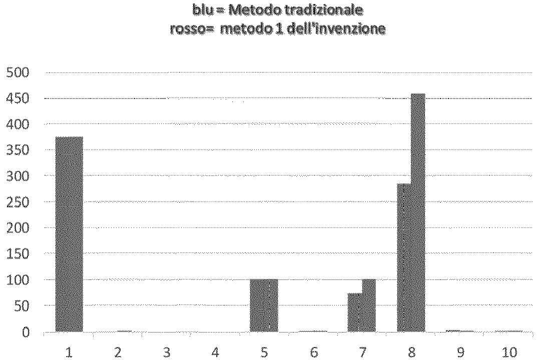

From the observation of Table 3 and the histogram, it is quite clear that the results of the two methods are almost identical. There are rare variations in the results which are likely due to experimental variability and/or to the fact that molecular biology procedures are notoriously more sensitive.

In view of the above it is quite evident that the method of the present invention, overcomes the drawbacks described in the introduction.

In fact, method 1 of the present invention advantageously exploits the combination of a qualitative analysis procedure (specifically the LAMP) and a quantitative analysis procedure (specifically the MPN count), in particular a statistical processing that allows having a quantitative result starting from the characteristic number NC, which essentially expresses a combination of positive and negative tubes, to determine the concentration of a bacterium of interest in a sample of environmental matrix, in a much faster time, using less material, at a lower cost and in conditions of lower biological risk.

Following Table 4 briefly summarizes some of the aspects revealed by the comparison of method 1 of the present invention with the traditional one.

**Table 4**

| **Traditional method** | **Method of the invention** |
|---|---|
| Analysis times between 6 and 10 days | Analysis times of approximately 1 day |
| Cost of analysis per sample € 180.09 | Cost of analysis per sample € 135.94 |
| High amount of special waste to be disposed of | Small amount of special waste to be disposed of |
| Non-ecological impact of the analysis | Ecological impact of the analysis |
| Exposure to chemical biological risk | Low exposure to chemical biological risk |
| Low sensitivity of the method | High sensitivity of the method |
| It requires large workspaces for the test execution | It requires reduced work spaces for the test execution |

As can be seen, the method of the present invention is from 6 to 10 times faster than the traditional method, thanks to the possibility of extracting, if present, the genome of the bacterium of interest from the analysed environmental matrix sample and the possibility of identifying and quantifying it through the combination of a qualitative analysis procedure, very fast compared to traditional identification procedures which require, as described in the introduction, the isolation of the bacterium on culture media, after having waited the time necessary for its multiplication, and a quantitative analysis procedure. The method of the present invention allows you to provide the concentration of the Salmonella spp. Bacterium in a dehydrated mud sample in less than 24 hours.

As will be noted, the method of the present invention advantageously also allows to obtain, thanks to the consultation of table 7, the uncertainty of the measurement, which makes it a completely reliable method.

Not only the procedure described above does make the method of the present invention easier to carry out than the traditional method but, advantageously, it also requires the use of a smaller amount of materials, which also occupy less volume than the traditional method and are therefore easier to be managed from a logistical point of view.

Furthermore, from an estimate of the costs required for the execution of the method, including not only the cost of the material but also the cost of man hours necessary for the execution of the method, it emerged that the method of the present invention allows to save about the 25% of the costs required by the traditional method.

Last but not least, the method of the present invention minimizes the biological risk. In fact, after cell lysis the pathogenic bacterium of interest is no longer infectious; while in the traditional method the biological risk is always present, since it requires, for its execution, the growth or multiplication of the bacterium itself.

In the foregoing the preferred embodiments were described and some modifications of the present invention were suggested, but it should be understood that those skilled in the art can make modifications and changes without departing from the relative scope of protection, as defined by the appended claims.

## Claims

1. Method (1) for determining the concentration of a bacterium of interest in one environmental matrix, the method comprising the following operational steps:
A. Preparing at least one primary suspension (2) of one sample of said environmental matrix;
B. Preparing one or more dilutions (21, 22, 23) of said at least one primary suspension (2) in respective first containers (31, 32, 33);
C. Preparing second containers (401, 402, 403; 411, 412, 413; 421, 422, 423; 431, 432, 433; 441, 442, 443) containing said at least one primary suspension (2) and said one or more dilutions (21, 22, 23);
D. extracting the genome of the content of each second container (401, 402, 403; 411, 412, 413; 421, 422, 423; 431, 432, 433; 441, 442, 443) into a respective third container (501, 502, 503; 511, 512, 513; 521, 522, 523; 531, 532, 533; 541, 542, 543);
E. Preparing at least one plurality of tubes (601, 602, 603; 611, 612, 613; 621, 622, 623; 631, 632, 633; 641, 642, 643), each tube of the plurality of tubes containing said genome contained in a respective third container (501, 502, 503; 511, 512, 513; 521, 522, 523; 531, 532, 533; 541, 542, 543);
F. Applying at least one qualitative analysis procedure to each tube (601, 602, 603; 611, 612, 613; 621, 622, 623; 631, 632, 633; 641, 642, 643), said qualitative analysis procedure being configured for detect the presence or absence of the genome of said bacterium of interest, thereby outputting for each tube (601, 602, 603; 611, 612, 613; 621, 622, 623; 631, 632, 633; 641, 642, 643) a corresponding index (101, 102, 103; I11, 112, 113; 121, 122, 123; 131, 132, 133; 141, 142, 143) of the presence or absence of said genome of said bacterium of interest;
G. Based on each index (101, 102, 103; I11, 112, 113; 121, 122, 123; 131, 132, 133; 141, 142, 143) of the presence or absence of said genome of said bacterium of interest, applying a quantitative analysis procedure, said quantitative analysis procedure being configured to determine the value of the most probable number of said bacteria of interest in said at least one sample of environmental matrix.

2. Method (1) according to claim 1, wherein said step A comprises:
- if said environmental matrix is a solid one, mechanically homogenize a preset amount of said sample of said environmental matrix with a sterile peptone saline solution, or
- if said environmental matrix is an aqueous one, preliminary filtering a preset amount of said sample of said aqueous environmental matrix, by using at least one filter, putting the filter used for said filtration in one container already containing another predetermined amount of said sample of said aqueous environmental matrix and shaking for a preset shaking interval.

3. Method (1) according to claim 1 or 2, wherein said step C comprises:
- inoculating a predefined amount of said primary suspension (2) and each subsequent dilution (21, 22, 23) of said primary suspension (2), in one or more respective second containers (401, 402, 403; 411, 412, 413; 421, 422, 423; 431, 432, 433; 441, 442, 443).

4. Method (1) according to claim 3, wherein if said environmental matrix is a solid one, each container contains at least one predefined amount of an averagely selective medium and said step C further comprises incubating each second container (401, 402, 403; 411, 412, 413; 421, 422, 423; 431, 432, 433; 441, 442, 443) at a preset incubation temperature, for a defined incubation interval, so that in each second container (401, 402, 403; 411, 412, 413; 421, 422, 423; 431, 432, 433; 441, 442, 443) the multiplication of said bacterium of interest, if present, is obtained.

5. Method (1) according to any previous claim, wherein, said step D comprises, for each second container (401, 402, 403; 411, 412, 413; 421, 422, 423; 431, 432, 433; 441, 442, 443):
- transferring one predefined amount of said primary suspension (2) or dilution (21, 22, 23) thereof, into said respective third container (501, 502, 503; 511, 512, 513; 521, 522, 523; 531, 532, 533; 541, 542, 543);
- adding to said third container (501, 502, 503; 511, 512, 513; 521, 522, 523; 531, 532, 533; 541, 542, 543) a predefined amount of at least one lysis buffer,
at a temperature at least equal or higher that a minimum temperature T_{MIN}, to break the cells therein contained; and
- centrifuging, said at least one third container (501, 502, 503; 511, 512, 513; 521, 522, 523; 531, 532, 533; 541, 542, 543) for at least one minimum time t_{MIN}.

6. Method (1) according to claim 5, wherein if said environmental matrix is an aqueous one, said step D, after transfer of said predefined amount of said primary suspension (2) or said dilution (21, 22, 23) thereof into said respective third container and before the addition in each third container of said predefined amount of at least one lysis buffer comprises:
- adding into each third container a predefined amount of an enzyme configured to degrade the genome of dead cells possibly therein contained.

7. Method (1) according to any previous claim, wherein said step E of preparing said at least one plurality of tubes (601, 602, 603; 611, 612, 613; 621, 622, 623; 631, 632, 633; 641, 642, 643), comprises:
- transferring a predefined amount of the content of each respective third container (501, 502, 503; 511, 512, 513; 521, 522, 523; 531, 532, 533; 541, 542, 543), optionally lower than 5 µl, more optionally equal to 3 µl, into a respective tube (601, 602, 603; 611, 612, 613; 621, 622, 623; 631, 632, 633; 641, 642, 643); and
- if not already present in each respective tube (601, 602, 603; 611, 612, 613; 621, 622, 623; 631, 632, 633; 641, 642, 643), adding at least one predefined amount of one amplification reagent of the genome of said bacterium of interest.

8. Method (1) according to any previous claim, wherein said quantitative analysis procedure comprises, for each tube (601, 602, 603; 611, 612, 613; 621, 622, 623; 631, 632, 633; 641, 642, 643):
- waiting for a predetermined amplification time interval, during which the amplification of said genome of said bacterium of interest possibly therein contained, optionally by means of a loop-mediated isothermal amplification technique, occurs;
- applying a detection technique of said genome thus amplified, and outputting a respective index (101, 102, 103; I11, 112, 113; 121, 122, 123; 131, 132, 133; 141, 142, 143) of presence or absence of said genome.

9. Method (1) according to claim 8, wherein said detection technique of said genome is a photometric technique and comprises, for each tube (611, 612, 613; 621, 622, 623; 631, 632, 633; 641, 642, 643; 651, 652, 653):
- detecting during said predetermined amplification time interval, at least one fluorescent light radiation emitted from each tube, as a result of the amplification of the genome triggered at the end of said step E; and
- outputting one corresponding index (101, 102, 103; I11, 112, 113; I11, 112, 113; I11, 112, 113; I11, 112, 113; I11, 112, 113) of presence or absence of said genome, based on the fact that said at least one fluorescent light radiation emitted by each tube so detected exceeds or not at least one predefined threshold value or it has one determined time pattern or not.

10. Method (1) according to any previous claims, wherein said quantitative analysis procedure comprises a statistical analytical technique of indirect counting of the most probable number of said bacteria of interest into said at least one sample of environmental matrix.

11. Method (1) according to claim 10, wherein said quantitative analysis procedure comprises:
- determining, on the basis of each index (101, 102, 103; I11, 112, 113; I11, 112, 113; I11, 112, 113; I11, 112, 113; I11, 112, 113) of presence or absence of said genome, one Characteristic Number (NC), according to the MPN value (most probable number) counting technique; and
- analytically determining, optionally by querying a database or consulting a consultation table (7), the value of the most probable number of said bacteria of interest per gram of sample (MPN/g) and/or the most probable number of said bacteria of interest per gram (MPN/g ss) of dry matter if said environmental matrix is solid, or the most probable number of said bacteria of interest per ml of sample (MPN/ml) and/or the most probable number of said bacteria of interest per 100 ml (MPN/100ml) of sample if said environmental matrix is aqueous, as a function of said Characteristic Number (NC), of said dilution ratio (R1, R2, R3) and of said percentage weight of dry matter into said sample (2).

12. Method (1) according to any previous claim, wherein said at least one bacterium of interest is Salmonella spp. or Legionella spp. or Legionella pneumophila.

13. Method (1) according to claim 12, wherein if said bacterium of interest is Salmonella spp., said step C comprises inoculating, in respective second containers (401, 402, 403; 411, 412, 413; 421, 422, 423; 431, 432, 433; 441, 442, 443) one preset amount of said at least one primary suspension (2) or one preset amount of at least one dilution (21, 22, 23) thereof into a predetermined amount of a Selenite-cystine based averagely selective medium and incubating at a temperature comprised between 36 ± 5 °C, optionally comprised between 36 ± 4 °C, more optionally between 36 ± 1 °C for 21 ± 3 hours.

14. Method (1) according to claim 12, wherein if said bacterium of interest is Salmonella spp. in said step D the cellular lysis of the content of said at least one third container (501, 502, 503; 511, 512, 513; 521, 522, 523; 531, 532, 533; 541, 542, 543) is carried out for at least one minimum lysis time interval tₘᵢₙ, optionally between 25 and 35 min, more optionally equal to 30 min at a temperature at least equal to or greater than a minimum temperature T_{MIN}, dove T_{MIN} is optionally equal to 60 °C, more optionally equal to 65 °C and wherein the centrifugation of said at least one third container (501, 502, 503; 511, 512, 513; 521, 522, 523; 531, 532, 533; 541, 542, 543) is performed for a minimum time interval t_{MIN} optionally lower than 10 minutes, more optionally equal to 5 minutes, at a speed between 7500/rpm and 9000/rpm, optionally equal to 8500 rpm.

15. Method (1) according to claim 12, when dependent upon claim 6, wherein if said bacterium of interest is Legionella spp. or Legionella pneumophila, in said step D after the transfer of said predefined amount of said primary suspension (2) or said dilution (21, 22, 23) thereof into said third container and before addition into said at least one third container of said predefined amount of at least one lysis buffer, one amount of said enzyme configured to degrade the genome of the dead cells possibly therein contained, optionally comprised between 45 µl and 55 µl, more optionally equal to 50 µl, is added into said at least one third container, and
the cellular lysis of the content of said at least one third container is carried out for at least one minimum lysis time interval tₘᵢₙ, optionally between 10 and 20 min, more optionally equal to 15 min at a temperature at least equal to or greater than a minimum temperature T_{MIN}, wherein T_{MIN} is optionally equal to 90°C, more optionally equal to 95°C and wherein the centrifugation of said at least one third container is performed for a minimum time interval t_{MiN} optionally lower than 10 minutes, more optionally equal to 5 minutes, at a speed between 7500/rpm and 9000/rpm, optionally equal to 8500 rpm.
